(19) **Europäisches Patentamt / European Patent Office / Office européen des brevets**

(11) **EP 2 791 346 B1**

(12) **FASCICULE DE BREVET EUROPEEN**

(45) Date de publication et mention
de la délivrance du brevet:
**09.09.2020 Bulletin 2020/37**

(21) Numéro de dépôt: **12813406.1**

(22) Date de dépôt: **29.11.2012**

(51) Int Cl.:
*C12P 7/10* (2006.01)     *C12P 19/02* (2006.01)
*C12P 19/14* (2006.01)     *C13K 1/02* (2006.01)
*G01N 11/00* (2006.01)     *G01N 27/02* (2006.01)
*G01N 3/26* (2006.01)

(86) Numéro de dépôt international:
**PCT/FR2012/000493**

(87) Numéro de publication internationale:
**WO 2013/088001 (20.06.2013 Gazette 2013/25)**

(54) **PROCÉDÉ DE PRODUCTION DE SUBSTRAT LIGNOCELLULOSIQUE LIQUÉFIÉ OPTIMISÉ**

VERFAHREN ZUR HERSTELLUNG EINES OPTIMIERTEN VERFLÜSSIGTEN LIGNOCELLULOSE-SUBSTRATES

PROCESS FOR PRODUCING AN OPTIMIZED LIQUEFIED LIGNOCELLULOSIC SUBSTRATE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.12.2011 FR 1103856**

(43) Date de publication de la demande:
**22.10.2014 Bulletin 2014/43**

(73) Titulaire: **IFP Energies nouvelles**
**92500 Rueil-Malmaison (FR)**

(72) Inventeurs:
• **LOURET, Sylvain**
**F-69003 Lyon (FR)**
• **ROUSSET, Romain**
**69007 Lyon (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
WO-A1-2011/157427     WO-A2-2013/082616
US-A- 4 409 329     US-A1- 2009 053 777
US-A1- 2010 297 705     US-A1- 2011 177 558

• **KYLE W. DUNAWAY ET AL: "Characterization of changes in viscosity and insoluble solids content during enzymatic saccharification of pretreated corn stover slurries", BIORESOURCE TECHNOLOGY, vol. 101, no. 10, 1 mai 2010 (2010-05-01), pages 3575-3582, XP055032685, ISSN: 0960-8524, DOI: 10.1016/j.biortech.2009.12.071**
• **DAVID B. HODGE ET AL: "Model-Based Fed-Batch for High-Solids Enzymatic Cellulose Hydrolysis", APPLIED BIOCHEMISTRY AND BIOTECHNOLOGY, vol. 152, no. 1, 1 janvier 2009 (2009-01-01), pages 88-107, XP055032441, ISSN: 0273-2289, DOI: 10.1007/s12010-008-8217-0 cité dans la demande**
• **BYUNG-HWAN UM ET AL: "A Comparison of Simple Rheological Parameters and Simulation Data for Zymomonas mobilis Fermentation Broths with High Substrate Loading in a 3-L Bioreactor", APPLIED BIOCHEMISTRY AND BIOTECHNOLOGY, vol. 145, no. 1-3, 1 mars 2008 (2008-03-01), pages 29-38, XP055032626, ISSN: 0273-2289, DOI: 10.1007/s12010-007-8105-z**
• **M. R. EHRHARDT ET AL: "Rheology of Dilute Acid Hydrolyzed Corn Stover at High Solids Concentration", APPLIED BIOCHEMISTRY AND BIOTECHNOLOGY, vol. 160, no. 4, 1 février 2010 (2010-02-01), pages 1102-1115, XP055032601, ISSN: 0273-2289, DOI: 10.1007/s12010-009-8606-z**

- SZIJÁRTÓ N ET AL: "Thermostable endoglucanases in the liquefaction of hydrothermally pretreated wheat straw", BIOTECHNOLOGY FOR BIOFUELS, BIOMED CENTRAL LTD, LONDON UK, vol. 4, no. 1, 2, 26 janvier 2011 (2011-01-26), XP021090911, ISSN: 1754-6834, DOI: 10.1186/1754-6834-4-2
- SHOEMAKER S P ET AL: "Enzymic activities of endo-1,4-beta-d-glucanases purified from Trichoderma viride", BBA ENZYMOLOGY, ELSEVIER, AMSTERDAM, NL, vol. 523, no. 1, 14 mars 1978 (1978-03-14) , pages 133-146, XP023355347, ISSN: 0005-2744, DOI: 10.1016/0005-2744(78)90016-5 [extrait le 1978-03-14]
- CLAUDIA C GEDDES ET AL: "Optimizing cellulase usage for improved mixing and rheological properties of acid-pretreated sugarcane bagasse", BIORESOURCE TECHNOLOGY, ELSEVIER BV, GB, vol. 101, no. 23, 10 juillet 2010 (2010-07-10), pages 9128-9136, XP028320925, ISSN: 0960-8524, DOI: 10.1016/J.BIORTECH.2010.07.040 [extrait le 2010-07-14]

**Description**

**DOMAINE DE L'INVENTION**

La présente invention concerne un procédé de production de substrat lignocellulosique liquéfié à partir de la biomasse ligno-cellulosique. Ledit substrat lignocellulosique liquéfié peut ensuite être utilisé dans diverses étapes ultérieures telles que par exemple dans une étape de fermentation pour la production d'alcools, ou pour la production d'intermédiaires pour la chimie.

**ART ANTÉRIEUR**

La mise au point de procédés économiquement viables de valorisation de la biomasse lignocellulosique est aujourd'hui un vaste sujet d'actualité. La raréfaction des ressources fossiles et la concurrence avec les ressources alimentaires conduisent à chercher de nouvelles voies pour la production de biocarburants et d'intermédiaires chimiques.

Depuis les années 1970, la transformation de la biomasse ligno-cellulosique après hydrolyse des polysaccharides constitutifs en sucres a fait l'objet de nombreux travaux.

La biomasse lignocellulosique se caractérise par une structure complexe constituée de trois principaux polymères, la cellulose, les hémicelluloses et la lignine, dont la proportion varie suivant les espèces de biomasse lignocellulosique. Une composition typique mais non limitative est la suivante : la cellulose à une teneur comprise entre 35 et 50%, les hémicelluloses qui sont des polysaccharides essentiellement constitués de pentoses et d'hexoses à une teneur comprise entre 20 et 30% et les lignines à une teneur comprise entre 15 et 25% du poids. La dégradation de la biomasse se révèle difficile car les polysaccharides de la paroi végétale (cellulose et hémicelluloses) sont intimement associés à de la lignine, qui confère aux parois leur rigidité.
De ces trois polymères, la cellulose est la principale source de sucres car elle est constituée de glucose, ce dernier pouvant être facilement valorisé.

De façon classique, les procédés de valorisation de la biomasse par voie biochimique comprennent plusieurs étapes. Une première étape est la collecte et le transport de la biomasse lignocellulosique dans un centre de transformation de la biomasse. La deuxième étape est le prétraitement ou préhydrolyse de la biomasse qui permet de rendre la cellulose accessible aux enzymes et ainsi de produire un substrat lignocellulosique prétraité. La troisième étape d'hydrolyse enzymatique permet, grâce à l'utilisation d'une solution d'enzymes cellulolytiques et hémicellulolytiques produites par des microorganismes et appelée cocktail enzymatique, la transformation de la cellulose en glucose. Ce glucose peut être ensuite valorisé par exemple en éthanol lors d'une quatrième étape de fermentation par, en général, la levure *Saccharomyces cerevisiae,* ou en mélange acétone, butanol, éthanol (ABE) par fermentation par la levure *Clostridium acetobutylcum.* Une cinquième étape de distillation permet ensuite de concentrer les molécules obtenues.

Ledit substrat lignocellulosique prétraité obtenu à l'issue de l'étape de prétraitement est un résidu solide composé essentiellement de cellulose et de lignine solide.
Dans toute la suite du texte, on exprime la concentration en substrat lignocellulosique prétraité en pourcentage poids de matière sèche. La matière sèche, exprimée en pourcentage poids, est le ratio de masse de l'échantillon obtenue après séchage à 105°C pendant 24 heures sur la masse initiale de l'échantillon.
Dans l'étape d'hydrolyse enzymatique, ledit substrat lignocellulosique prétraité doit être mélangé avec une solution liquide contenant les enzymes cellulolytiques et hémicellulolytiques. L'objectif étant d'obtenir une concentration élevée en sucres, l'étape d'hydrolyse enzymatique doit se faire à des concentrations élevées en substrats lignocellulosique prétraité, c'est-à-dire à haute teneur en matière sèche. Le mélange intime avec ladite solution liquide contenant les enzymes cellulolytiques et hémicellulolytiques s'avère donc difficile.

L'étape d'hydrolyse enzymatique peut donc être réalisée avec un substrat lignocellulosique prétraité dilué par ajout d'eau de façon à homogénéiser le milieu réactionnel. La dilution à l'eau dudit substrat lignocellulosique présente l'inconvénient de diluer également les sucres et les oligomères de sucres obtenus à l'issue de l'étape d'hydrolyse enzymatique. Afin de palier ce problème de dilution et de pouvoir assurer une concentration en sucre intéressante et viable, des mises en oeuvres spécifiques peuvent être envisagées : l'alimentation dudit substrat lignocellulosique en mode "fed-batch", défini plus bas, permet de préparer un fond de réacteur suffisamment dilué pour pouvoir initier la réaction d'hydrolyse enzymatique et assurer un bon mélange. Au fur et à mesure de l'avancée de la réaction, le mélange devenant de plus en plus liquide, il est possible d'ajouter du substrat frais de manière à monter sa concentration. En procédant de la sorte, il est alors possible d'atteindre des concentrations en substrat élevé et avantageusement compris entre 20 et 30 % poids de matière sèche.
Ce début d'hydrolyse à haute teneur en matière sèche pose des problèmes de mélange et d'homogénéisation. Le milieu réactionnel est très pâteux et visqueux ce qui demande une agitation spécifique beaucoup plus complexe que celle nécessaire en fin d'hydrolyse où le mélange réactionnel est devenu plus liquide. C'est pourquoi, il semble pertinent de séparer cette hydrolyse en deux étapes :

- La liquéfaction correspond au début d'hydrolyse durant laquelle le milieu réactionnel est visqueux et demande un système d'agitation complexe et une énergie d'agitation importante. En revanche, le temps de séjour est court ce qui permet de limiter les volumes à installer avec une telle agitation.
- L'étape suivante correspond à la saccharification dans le cas où l'on prolonge l'hydrolyse classiquement ou à la SSF (simultaneous saccharification and fermentation) dans le cas où l'on introduit des levures pour fermenter les sucres tout en hydrolysant. Cette étape demande une agitation plus simple, moins d'énergie, mais un temps de séjour plus long et donc un volume plus important.

Les travaux de recherche de la Demanderesse l'ont amené à s'intéresser plus particulièrement à l'étape de liquéfaction afin de mieux suivre son avancée et d'optimiser son pilotage.

La présente invention propose donc de suivre la mesure d'une caractéristique rhéologique du milieu réactionnel de manière à optimiser la liquéfaction, tout en jouant sur les ajouts de substrat lignocellulosique, d'enzyme ou sur la dilution.

Un objectif du procédé selon la présente invention est donc de fournir un procédé de production de substrat lignocellulosique liquéfié par réaction enzymatique permettant l'obtention d'un substrat lignocellulosique liquéfié présentant une rhéologie compatible avec son transfert vers l'étape placée en aval ainsi qu'avec son agitation dans la réaction suivante.

La demande de brevet US 2010/0255554 décrit un procédé d'hydrolyse de la biomasse lignocellulosique en mode "fed-batch" dans lequel les paramètres de fonctionnement du procédé sont ajustés en contrôlant le volume du réacteur et/ou la fréquence d'addition de la charge biomasse lignocellulosique prétraitée et éventuellement l'ajout d'enzymes, et le volume et/ou la concentration en sucres produits dans le réacteur. En particulier, la charge biomasse lignocellulosique prétraitée est ajouté de manière séquentielle dans le réacteur, à chaque fois que 70 à 90% de conversion théorique de la cellulose en glucose est atteinte dans le milieu réactionnel. C'est donc le suivi de la mesure de la concentration en sucres produits qui constitue le critère d'optimisation du procédé. Cette méthode requière donc de connaître le taux de conversion théorique pour un substrat donné, ce qui, compte tenu de la variabilité des substrats potentiellement traités est difficilement applicable.

Hodge et al., Model-Based Fed-Batch for High-Solids Enzymatic Cellulose Hydrolysis, Appl.Biochem Biotechnol. (2009) 152:88-107 décrit procédé d'hydrolyse de la biomasse lignocellulosique en mode "fed-batch" basé sur la modélisation de la réaction d'hydrolyse. Il est donc nécessaire de déterminer, pour chaque substrat ou mélange de substrats, leur comportement au cours de l'hydrolyse afin de déterminer la meilleure stratégie d'addition de charge dans le réacteur.

Rosgaard et al., Effects of Substrate Loading on Enzymatic Hydrolysis and Viscosity of Pretreated Barley Straw, Appl.Biochem Biotechnol. (2007) 143:27-40 examine l'effet de différentes stratégies d'addition de substrat lignocellulosique prétraité sur les rendements en sucres de l'hydrolyse enzymatique. Elle décrit le phénomène par ailleurs bien connu de chute de viscosité au fur et à mesure de l'avancement de la réaction. L'enseignement de cet article ne permet pas de généraliser la stratégie d'alimentation du substrat et/ou de la solution enzymatique en cas de changement de substrat et ne tient pas compte des contraintes mécaniques de l'agitation.

La demande de brevet US2010/330638A décrit une alimentation en mode « Fed-batch » de l'étape d'hydrolyse enzymatique, en indiquant que des tests permettent de déterminer la quantité de biomasse qui peut être ajoutée à chaque batch. Il est donc nécessaire d'effectuer des tests préalables à l'étape d'hydrolyse enzymatique lors de chaque changement de type de substrat.

Contrairement à l'état de l'art antérieur, la présente invention propose un procédé de production de substrat lignocellulosique liquéfié dans lequel est effectué un suivi de la mesure d'une caractéristique rhéologique du milieu réactionnel de manière à optimiser la liquéfaction.

En particulier, la présente invention propose un procédé de transformation d'un substrat lignocellulosique comprenant les étapes suivantes :

1) une étape de liquéfaction du substrat dans laquelle on met en contact, sous agitation au moyen d'un moteur, 10 à 40% poids de matière sèche de substrat lignocellulosique prétraité avec de l'eau et avec des enzymes à une concentration comprise entre 0,1 à 60 mg d'enzymes par grammes de cellulose pendant une durée comprise entre 1 et 24 heures, dans laquelle on mesure au cours du temps au moins la valeur de la puissance électrique consommée par le moteur du système d'agitation et, si l'on détecte une diminution au cours du temps de ladite valeur, on effectue une étape a) suivante :

a) on augmente le débit d'alimentation en substrat lignocellulosique prétraitée, avec ou sans modification du débit d'enzymes et/ou d'eau,

et si l'on détecte une augmentation au cours du temps de ladite valeur, on effectue une étape b) suivante :
b) on augmente le débit d'alimentation en eau et/ou en enzymes, avec ou sans modification du débit en substrat lignocellulosique prétraitée

la puissance électrique consommée par le moteur du système d'agitation ramenée à la masse du volume réactionnel restant comprise 0,5 et 2kW/tonne,

2) une étape de saccharification du substrat lignocellulosique liquéfié issu de l'étape 1), ladite étape de saccharification étant réalisée en présence d'un microorganisme alcooligène selon un procédé de saccharification et de fermentation simultanées dit procédé SSF.

Un avantage de la présente invention est de fournir un procédé de production de substrat lignocellulosique liquéfié pouvant être conduit de manière automatique et indépendamment de la nature du substrat traité.

Un autre avantage de la présente invention est de fournir un procédé de production de substrat lignocellulosique liquéfié ne nécessitant pas de caractérisation préalable du substrat traité, et permettant ainsi une plus grande flexibilité de l'installation et une facilité d'utilisation accrue.

La présente invention ne nécessite pas non plus de développement de modèle réactionnel et reste donc pertinente quelque soit le type de substrat ou de cocktail enzymatique utilisé.

Un autre avantage de la présente invention est de fournir un procédé de production de substrat lignocellulosique liquéfié permettant un suivi et une adaptation simple à l'évolution du milieu réactionnel ne nécessitant pas de mesures complexes telles que par exemple la concentration en sucres dans le milieu.

Un autre document de l'art antérieur, US4409329, décrit un procédé de saccharification d'un substrat cellulosique avec broyage du substrat pendant la réaction, dans lequel la viscosité du milieu réactionnel est mesurée pour contrôler le taux d'addition de substrat, d'enzymes et/ou d'eau.

## DESCRIPTION DÉTAILLÉE DE L'INVENTION

Le substrat lignocellulosique prétraitée utilisé dans le procédé selon la présente invention est avantageusement obtenu par des procédés classiques de prétraitement de la biomasse lignocellulosique telles que par exemple les cuissons acides, les cuissons alcalines, l'explosion à la vapeur, les procédés organosolv, etc. Les prétraitements de type acide en condition douce et par explosion à la vapeur sont les mieux adaptés. Ils permettent une bonne accessibilité de la cellulose à l'hydrolyse.

Le procédé selon la présente invention est avantageusement un procédé de liquéfaction. Ledit procédé est avantageusement réalisé dans un réacteur de forme cylindrique présentant un ratio hauteur/diamètre avantageusement compris entre 1 et 3.

Ledit réacteur permet de traiter des milieux visqueux présentant une viscosité variable et ainsi de mettre en œuvre des taux de matière sèche en substrat lignocellulosique pouvant atteindre les 40% poids. Les taux de matière sèche élevés et la viscosité importante du milieu réactionnel requiert que le réacteur soit équipé d'un agitateur permettant un bon contact entre enzyme et substrat et une bonne homogénéité. Classiquement, l'agitateur choisi doit pouvoir traiter des écoulements laminaires. Des agitateurs larges, voire raclant la paroi du réacteur avec des vitesses de rotation modérée et exerçant une action de malaxage et pétrissage sont préférés. Un exemple d'agitateur particulièrement adapté est le Paravisc (EKATO) qui permet aussi l'ajout d'une contre-pale cassant les mouvements d'ensemble.

Conformément à l'invention, le substrat lignocellulosique prétraitée est mise en contact dans le procédé selon la présente invention à une concentration comprise entre 10 à 40% poids de matière sèche de substrat lignocellulosique prétraitée, de préférence à une concentration comprise entre 16 et 30% poids de matière sèche et de manière préférée comprise entre 18 et 24% poids de matière sèche.

Conformément à l'invention, les enzymes sont mises en contact dans le procédé selon la présente invention à une concentration comprise entre 0,1 à 60 mg d'enzymes par grammes de cellulose, de préférence à une concentration comprise entre 5 et 30 mg d'enzymes par grammes de cellulose et de manière préférée comprise entre 10 et 20 mg d'enzymes par grammes de cellulose.

Conformément à l'invention, la durée de mise en contact est comprise entre 1 et 24 heures, de préférence entre 2 et 12 heures et de manière préférée entre 4 et 8 heures.

Ledit procédé selon la présente invention se caractérise en ce qu'on réalise une mesure au cours du temps d'au moins la valeur de l'une des caractéristiques rhéologiques du milieu réactionnel.

Lesdites caractéristiques rhéologiques du milieu réactionnel sont avantageusement choisies parmi la viscosité du milieu réactionnel, le couple de torsion de l'arbre du système d'agitation et la puissance électrique consommée par le moteur. La puissance électrique consommée par le moteur est notée $P_{elec}$.

Durant le procédé selon l'invention, c'est à dire durant la liquéfaction, la viscosité du milieu réactionnel, le couple de torsion de l'arbre du système d'agitation et la puissance électrique consommée par le moteur sont des caractéristiques rhéologiques de suivi du substrat lignocellulosique produit qui présentent plusieurs intérêts. En effet, lesdites caractéristiques, viscosité, couple et puissance, sont reliées entre elles. La puissance électrique consommée par le moteur $P_{elec}$ est liée à la puissance mécanique $P_{méca}$ entraînant l'arbre d'agitation.

La puissance électrique consommée par le moteur est un paramètre classiquement mesuré et suivi sur les installations pilote ou industrielle.

Les formules suivantes définissent les relations entre les différents paramètres :

$P_{méca}$ = f ($P_{elec}$), f étant une caractéristique de conception du moteur et étant donnée par le constructeur du moteur. $P_{méca}$ = 2πN*C dans laquelle :

N est la vitesse agitation en tour par seconde,
C est le couple en N.m,
et $P_{méca}$ est la puissance en watt.

En agitation on a la relation suivante :
$P_{méca} = \rho N_p N^3 D^5$

$\rho$ est la densité du milieu réactionnel en kg.m$^{-3}$
D est le diamètre externe de l'agitateur en m,
$N_p$ est une caractéristique de l'agitateur dépendant de la géométrie de la cuve et du régime d'écoulement.

En régime d'écoulement laminaire, on a la relation suivante :

$$N_p = A/Re \text{ d'où } P_{méca} = \rho A N^3 D^5 / Re$$

avec A étant une constante du système d'agitation et Re le nombre de Reynolds et Re = $\rho ND^2/\overline{\mu}$,
$\overline{\mu}$ étant la viscosité dynamique moyenne mesurée en Pascal seconde (Pa.s) du milieu réactionnel avec $\overline{\mu}$ = $P_{méca}/(AN^2D^3) = 2\pi C/(AD^3N)$

Si la viscosité et le couple de torsion de l'arbre du système d'agitation sont des mesures facilement accessibles à petite échelle, la puissance électrique consommée par le moteur $P_{elec}$ est la grandeur la plus facilement mesurable à échelle industrielle. De manière très préférée, ledit procédé selon la présente invention se caractérise en ce qu'on réalise une mesure au cours du temps de la puissance électrique consommée par le moteur.

L'évolution de la puissance électrique consommée par le moteur est donc corrélée à la liquéfaction du substrat lignocellulosique prétraité et à l'avancée de la réaction. Si la puissance baisse, cela signifie que le substrat lignocellulosique prétraité s'est liquéfié et que l'on peut ajouter du substrat frais. Si la puissance augmente, cela signifie que l'on vient de rajouter du substrat frais et qu'il faut laisser encore du temps pour retrouver le niveau de puissance avant ajout.

Conformément à l'invention, la mesure desdites caractéristiques rhéologiques du milieu réactionnel est suivie de manière à ce que si on détecte une diminution au cours du temps de ladite valeur, on effectue une étape a) suivante :

a) on augmente le débit d'alimentation en substrat lignocellulosique prétraitée, avec ou sans modification du débit d'enzymes et/ou d'eau,

et en ce que si on détecte une augmentation au cours du temps de ladite valeur, on effectue une étape b) suivante :
b) on augmente le débit d'alimentation en eau et/ou en enzymes, avec ou sans modification du débit en substrat lignocellulosique prétraitée.

Dans le cas où ladite valeur est stable au cours du temps, on maintient avantageusement les débits en substrat, en enzyme et en eau constants.

Selon un mode de réalisation préféré, le procédé de production de substrat lignocellulosique liquéfié par réaction enzymatique selon l'invention opère avantageusement en mode "fed batch" selon la terminologie anglo-saxonne. Dans ce cas, ledit procédé est mis en œuvre dans un réacteur à alimentation continue au cours de laquelle aucun soutirage du contenu du réacteur n'est effectué.

Selon un autre mode de réalisation préféré, le procédé de production de substrat lignocellulosique liquéfié par réaction enzymatique selon l'invention opère avantageusement en mode "chemostat" selon la terminologie anglo-saxonne. Dans ce cas, ledit procédé est mis en oeuvre dans un réacteur à alimentation continue au cours de laquelle une fraction du volume réactionnel est soutirée de manière à maintenir la masse du volume réactionnel constante.

Selon ledit mode de réalisation préféré dans lequel ledit procédé est mis en oeuvre dans un réacteur à alimentation continue et dans le cas où l'on détecte une diminution au cours du temps de ladite valeur, ladite étape a) est mise en œuvre selon un premier mode de réalisation dans lequel on augmente le débit d'alimentation en substrat lignocellulosique prétraité tout en diminuant le débit d'enzyme et d'eau de manière à conserver un volume réactionnel constant. Dans ce cas, le débit de sortie reste constant.

Ladite étape a) peut également avantageusement être mise en œuvre selon un deuxième mode de réalisation dans lequel on augmente le débit en substrat lignocellulosique prétraité ainsi que les débits d'enzyme et d'eau de manière à conserver la concentration en enzyme et le taux de matière sèche constant. Le débit de sortie est alors être augmenté pour maintenir le volume réactionnel constant. Dans ce cas, le temps de réaction de la liquéfaction diminue.

Ladite étape a) peut également avantageusement etre mise en œuvre selon un troisième mode de réalisation dans lequel on augmente le débit en substrat lignocellulosique prétraité tout en maintenant les débits d'enzyme et d'eau constants. Le débit de sortie est alors être augmenté pour maintenir le volume réactionnel constant. Dans ce cas, le temps de réaction de la liquéfaction diminue.

Selon ledit mode de réalisation préféré dans lequel ledit procédé est mis en œuvre dans un réacteur à alimentation continue et dans le cas où l'on détecte une augmentation au cours du temps de ladite valeur, ladite étape b) est mise en œuvre selon un premier mode de réalisation dans lequel on diminue le débit en substrat lignocellulosique prétraité tout en augmentant le débit d'enzyme et d'eau de manière à conserver un volume réactionnel constant. Le débit de sortie reste constant.

Ladite étape b) peut également avantageusement etre mise en œuvre selon un deuxième mode de réalisation dans lequel on diminue le débit en substrat lignocellulosique prétraité ainsi que les débits d'enzyme et d'eau de manière à conserver la concentration en enzyme et le taux de matière sèche constant. Le débit de sortie est alors réduit pour maintenir le volume réactionnel constant. Dans ce cas, le temps de réaction de la liquéfaction augmente.

Ladite étape b) peut également avantageusement etre mise en œuvre selon un troisième mode de réalisation dans lequel on diminue le débit en substrat lignocellulosique prétraité tout en maintenant les débits d'enzyme et d'eau constants. Le débit de sortie est également augmenté pour maintenir le volume réactionnel constant. Dans ce cas, le temps de réaction de la liquéfaction augmente.

De préférence, les étapes a) et b) sont mises en œuvre par un opérateur ou automatisée tel que par exemple par un logiciel de contrôle.

Le procédé selon la présente invention opère avantageusement à une température comprise entre 40 et 60°C, et de préférence entre 45 et 55°C, à un pH compris entre 4 et 6, et de préférence compris entre 4,5 et 5 et à pression atmosphérique.

La vitesse d'agitation dépend de la taille du réacteur et de l'agitateur.

Dans le cas où l'on mesure au cours du temps la valeur de la puissance électrique consommée par le moteur, ladite puissance électrique consommée par le moteur ramenée à la masse du volume réactionnel reste avantageusement comprise entre 0,05 et 4kW/tonne et de préférence entre 0,5 et 2kW/tonne.

La température est également un paramètre sur lequel on peut jouer.

Si la valeur de l'une des caractéristiques rhéologiques du milieu réactionnel que l'on mesure au cours du temps est élevée et que les manières d'opérer selon l'étape b) ne permettent pas la diminuer, on peut augmenter la température pendant une durée inférieure à 5h, de préférence inférieure à 2h. Dans ce cas, la température est augmentée de sorte qu'elle reste comprise entre 40 et 60°C et de préférence entre 45 et 55°C.

Cette hausse de température accélère temporairement la réaction, mais a pour conséquence de désactiver les enzymes plus rapidement. Elle est équivalent à une hausse temporaire de la concentration en enzyme.

Si la valeur de l'une des caractéristiques rhéologiques du milieu réactionnel que l'on mesure au cours du temps est faible et que les manières d'opérer selon l'étape a) ne permettent pas de l'augmenter, on peut diminuer la température pendant une durée inférieure à 5h, et de préférence inférieure à 2h. Dans ce cas, la température est diminuée de sorte qu'elle reste comprise entre 40 et 60°C et de préférence entre 45 et 55°C.

La réaction est ralentie, mais les enzymes se désactivent moins vite. Cette action est équivalente à une baisse temporaire de la concentration en enzyme.

Le procédé selon l'invention permet donc l'obtention d'un substrat lignocellulosique présentant une rhéologie facilitant son pompage ainsi que son agitation.

Le substrat lignocellulosique liquéfié obtenu à l'issue du procédé selon l'invention peut donc être facilement transféré dans un autre réacteur pour toute étape ultérieure.

Le procédé selon l'invention peut donc être avantageusement suivi de toute étape ultérieure permettant la transformation du substrat lignocellulosique liquéfié obtenu à l'issue du procédé selon l'invention.

Le procédé selon l'invention peut donc être avantageusement suivi d'une étape de saccharification. Ladite étape de saccharification permet la production de sucre par hydrolyse enzymatique et est poursuivie dans des conditions similaires au procédé selon l'invention, c'est-à-dire à la liquéfaction, mais avec un système d'agitation plus simple et une puissance d'agitation plus faible.

Selon un mode de réalisation préféré, l'étape de saccharification est avantageusement réalisée en présence d'un microorganisme alcooligène de manière à obtenir un moût de fermentation. Dans ce cas, la saccharification et la fermentation alcoolique sont réalisées en une seule étape selon un procédé de saccharification et de fermentation simultanées dit procédé SSF.

Le procédé selon l'invention peut également être avantageusement suivi d'une étape production d'un mélange acétone, butanol, éthanol (ABE), ladite étape étant avantageusement réalisée en présence de la levure *Clostridium acetobutylcm.*

Le procédé selon l'invention peut également être avantageusement suivi d'une étape production d'intermédiaires pour la chimie.

L'exemple de fonctionnement ci après permet d'illustrer le procédé selon l'invention.

## EXEMPLES

Exemple N°1 selon l'invention : procédé de liquéfaction en mode Fed-batch

L'exemple N°1 traite d'un procédé de liquéfaction effectuée dans un mode fed-batch. Le principe consiste à préparer un fond de cuve avec une partie du substrat lignocellulosique prétraité à liquéfier. Cela permet de commencer la réaction avec un substrat lignocellulosique prétraité dilué permettant un mélange. Au fur et à mesure de la liquéfaction et de la chute de viscosité du milieu on peut faire des ajouts de substrat frais afin de compléter le volume du réacteur et d'atteindre une matière sèche importante.

Le substrat lignocellulosique prétraité considérée est de la paille de blé. Un prétraitement adapté pour rendre la paille réactive à l'hydrolyse est l'explosion à la vapeur continue. Au préalable, une étape de broyage et une imprégnation acide sont requis. Une grille de 10 à 20 mm est amplement suffisante. L'imprégnation acide se fait dans une solution d'acide sulfurique avec un taux de matière sèche de 10%. La dose d'acide est de 0.50% poids d'acide sulfurique par rapport à la matière sèche. La biomasse est égouttée et introduite dans le réacteur d'explosion à la vapeur.

L'explosion à la vapeur est effectuée dans les conditions opératoire suivantes :

- T = 200°C
- P = 16 bars
- Temps de séjour : 5 min
- Taux de matière sèche dans le réacteur : 20 -25%

La paille prétraitée est ensuite ramenée à pH 4.8 avec une solution concentré de potasse KOH puis peut être pressée pour atteindre une matière sèche de 35%. La pureté en cellulose du substrat obtenue après ce type de prétraitement est communément d'environ 50% poids.

L'étape de liquéfaction est effectué dans un réacteur de 4m$^3$ équipé d'un agitateur de type Paravisc d'EKATO. Le cocktail enzymatique utilisé est la Cellic-CTec2 de Novozyme. La solution enzymatique a une concentration en protéine de 200 g/L et une densité de 1,2 environ.

Les conditions opératoires du fed-batch sont les suivantes :

- Taux de matière sèche du milieu réactionnel initial : 10%
- Taux de matière sèche du milieu réactionnel final : 20%
- Concentration d'enzyme : 20 g de solution enzymatique / kg de cellulose
- Vitesse d'agitation Paravisc : 15 tours par minute
- pH = 4.8, régulé avec de la potasse concentrée
- Température = 50°C, régulée
- Ajouts de substrats lignocellulosique prétraité : 5 ajouts de 320 kg chacuns

Initialement vide, le réacteur est rempli par 1675 kg d'eau auxquels on ajoute 686kg de substrat lignocellulosique prétraitée. On met le réacteur en chauffe pour atteindre la consigne de 50°C. Une fois cette consigne atteinte, on introduit la totalité des enzymes requises soit 40kg de solution enzymatique. La réaction commence alors avec une matière sèche de 10% et un volume réactionnel d'environ 2.4m$^3$. Nous appellerons t0 l'instant d'ajout des enzymes correspondant au début de réaction. A t0, la puissance électrique consommée par le moteur d'agitation ramenée à la masse de milieu réactionnel est de 4.0 kW/tonnes. La réaction se déroulant, la puissance électrique consommée par le moteur ramenée à la masse de milieu réactionnel chute et finit par atteindre 2.0 kW/tonnes. On fait alors un premier ajout de 320kg de substrat faisant remonter la puissance électrique consommée par le moteur ramenée à la masse de milieu réactionnel. On répète cette procédure jusqu'à avoir effectué les 5 ajouts. Généralement, le dernier ajout est effectué environ à t0 + 3h. On laisse la réaction se dérouler encore 3h de manière à faire chuter la viscosité avant de pouvoir aller à l'étape ultérieure ou de passer en mode continu. On peut espérer atteindre une puissance électrique consommée par le moteur ramenée à la masse de milieu réactionnel de 0.5 kW/tonnes en fin de liquéfaction.

Exemple N°2 selon l'invention : procédé de liquéfaction en mode continu à partir d'un fed-batch

L'exemple N°2 traite d'un procédé de liquéfaction effectué en mode continu à partir d'un mode fed-batch expliqué dans l'exemple N°1. Les réactifs et conditions opératoires du mode "fed batch" restent les mêmes. La transition du mode fed-batch vers le mode continu s'effectue une fois que le réacteur de 4m$^3$ est plein et que la puissance électrique consommée par le moteur ramenée à la masse de milieu réactionnel est de 1.0 kW/tonnes. A ce moment là, le produit est soutiré en continu à un débit permettant de conserver un volume réactionnel constant. Pour un temps de séjour de 4h, cela correspond à un débit de 1 tonne par heure. A ce même moment, du substrat, de l'eau et des enzymes sont injectés avec un débit général identique pour maintenir le la masse et le volume constant dans le réacteur. Le débit massique de chaque flux est alors le suivant :

- Eau : 419kg/h
- Solution enzymatique : 10kg/h
- Substrat : 571kg/h

La liquéfaction continue commence alors et trois cas de figure peuvent se présenter :

- la puissance électrique consommée par le moteur reste constante, auquel cas on ne modifie pas les débits de substrat, de solution enzymatique ou d'eau
- la puissance électrique consommée par le moteur diminue, auquel cas on peut :

  ◦ augmenter le débit de substrat en diminuant celui d'enzyme et d'eau pour conserver un volume constant. Le débit de sortie reste constant. Ex : 650kg/h de substrat, 9kg/h d'enzyme, 341kg/h d'eau et un débit de sortie de 1000kg/h
  ◦ augmenter le débit de substrat en maintenant les débits d'enzyme et d'eau constant. Le débit de sortie doit aussi être augmenter pour maintenir le volume constant. Ex : 650kg/h de substrat, 10kg/h d'enzyme, 419kg/h d'eau et un débit de sortie de 1079kg/h.

- la puissance électrique consommée par le moteur augmente, auquel cas on peut :

  ◦ diminuer le débit de substrat en augmentant celui d'enzyme et d'eau pour conserver un volume constant. Le débit de sortie reste constant. Ex : 500kg/h de substrat, 12kg/h d'enzyme, 488kg/h d'eau et un débit de sortie de 1000kg/h
  ◦ diminuer le débit de substrat en maintenant les débits d'enzyme et d'eau constant. Le débit de sortie doit aussi être augmenter pour maintenir le volume constant. Ex : 500kg/h de substrat, 10 kg/h d'enzyme, 419kg/h d'eau et un débit de sortie de 929kg/h.

Suivant les évolutions de la puissance électrique consommée par le moteur ramenée à la masse de milieu réactionnel, il est possible de piloter ainsi le procédé de liquéfaction afin de maintenir un point de fonctionnement caractérisé par la viscosité à un cisaillement donnée. Le raisonnement est similaire avec un suivi en puissance d'agitation ou en couple.

Exemple 3 selon l'invention : Modes de réalisation de l'étape a)

L'exemple n°3 traite des différents modes de réalisation de l'étape a). Le réacteur de liquéfaction fonctionne de manière stable en régime continu et les débits en substrat, eau et enzyme sont respectivement de 40 kg/h, 59 kg/h et 1 kg/h comme indiqué dans le Tableau , colonne « Cas nominal ».
On détecte au cours du temps une diminution de la caractéristique rhéologique mesurée.
En partant du cas nominal, on augmente le débit de substrat de 40 à 50 kg/h, et on diminue les débits d'eau et d'enzyme respectivement de 59 à 49,5 kg/h et de 1 à 0,5 kg/h. Le débit total est conservé et la caractéristique rhéologique mesurée est de nouveau à sa valeur nominale. Ce mode de réalisation est présenté dans le Tableau , colonne « Cas a)1) ».
En partant du cas nominal, on augmente le débit de substrat de 40 à 50 kg/h, et on augmente les débits d'eau et d'enzyme respectivement de 59 à 73,75 kg/h et de 1 à 1,25kg/h.
Par rapport au cas nominal, le ratio débit d'eau sur débit de substrat et le ratio débit d'enzyme sur débit de substrat est conservé. La caractéristique rhéologique mesurée est de nouveau à sa valeur nominale. Ce mode de réalisation est présenté dans le Tableau , colonne « Cas a)2) ».
En partant du cas nominal, dans un troisième mode de réalisation, on augmente le débit de substrat de 40 à 50 kg/h, et on conserve les débits d'eau et d'enzyme constant. La caractéristique rhéologique mesurée est de nouveau à sa valeur nominale. Ce mode de réalisation est présenté dans le Tableau , colonne « Cas a)3) ».

**Tableau 1 Modes de réalisation de l'étape a) - débits en kg/h**

| Données | Cas nominal | Cas a)1) | Cas a)2) | Cas a)3) |
|---|---|---|---|---|
| Débit de substrat | 40 | 50 | 50 | 50 |
| Débit d'eau | 59 | 49.5 | 73.75 | 59 |
| Débit d'enzyme | 1 | 0.5 | 1.25 | 1 |
| Débit total | 100 | 100 | 125 | 110 |
| Effets | | Dose et taux MS diminuent | Dose et taux MS constants | Dose et taux MS diminuent |

Exemple 4 selon l'invention : Modes de réalisation de l'étape b)

L'exemple n°4 traite des différents modes de réalisation de l'étape b). Le réacteur de liquéfaction fonctionne de manière stable en régime continu et les débits en substrat, eau et enzyme sont respectivement de 40 kg/h, 59 kg/h et 1 kg/h comme indiqué dans le Tableau , colonne « Cas nominal ».
On détecte au cours du temps une augmentation de la caractéristique rhéologique mesurée.
En partant du cas nominal, dans un premier mode de réalisation, on diminue le débit de substrat de 40 à 30 kg/h, et on augmente les débits d'eau et d'enzyme respectivement de 59 à 68,5 kg/h et de 1 à 1,5 kg/h. Le débit total est conservé et la caractéristique rhéologique mesurée est de nouveau à sa valeur nominale. Ce mode de réalisation est présenté dans le Tableau , colonne « Cas b)1) ».
En partant du cas nominal, dans un deuxième mode de réalisation, on diminue le débit de substrat de 40 à 30 kg/h, et on diminue les débits d'eau et d'enzyme respectivement de 59 à 44,25 kg/h et de 1 à 0,75 kg/h.
Par rapport au cas nominal, le ratio débit d'eau sur débit de substrat et le ratio débit d'enzyme sur débit de substrat est conservé. La caractéristique rhéologique mesurée est de nouveau à sa valeur nominale. Ce mode de réalisation est présenté dans le Tableau , colonne « Cas b)2) ».
En partant du cas nominal, dans un troisième mode de réalisation, on diminue le débit de substrat de 40 à 30 kg/h, et on conserve les débits d'eau et d'enzyme constant. La caractéristique rhéologique mesurée est de nouveau à sa valeur nominale. Ce mode de réalisation est présenté dans le Tableau , colonne « Cas b)3) ».

**Tableau 2 Modes de réalisation de l'étape b) - débits en kg/h**

| Données | Cas nominal | Cas b)1) | Cas b)2) | Cas b)3) |
|---|---|---|---|---|
| Débit de substrat | 40 | 30 | 30 | 30 |
| Débit d'eau | 59 | 68.5 | 44.25 | 59 |
| Débit d'enzyme | 1 | 1.5 | 0.75 | 1 |
| Débit total | 100 | 100 | 75 | 90 |
| Effets | | Dose et taux MS augmentent | Dose et taux MS constants | Dose et taux MS augmentent |

## Revendications

1. Procédé de transformation d'un substrat lignocellulosique comprenant les étapes suivantes :

   1) une étape de liquéfaction du substrat dans laquelle on met en contact, sous agitation au moyen d'un moteur, 10 à 40% poids de matière sèche de substrat lignocellulosique prétraité avec de l'eau et avec des enzymes à une concentration comprise entre 0,1 à 60 mg d'enzymes par grammes de cellulose pendant une durée comprise entre 1 et 24 heures, dans laquelle on mesure au cours du temps au moins la valeur de la puissance électrique consommée par le moteur du système d'agitation et, si l'on détecte une diminution au cours du temps de ladite valeur, on effectue une étape a) suivante :

a) on augmente le débit d'alimentation en substrat lignocellulosique prétraitée, avec ou sans modification du débit d'enzymes et/ou d'eau,

et si l'on détecte une augmentation au cours du temps de ladite valeur, on effectue une étape b) suivante :
b) on augmente le débit d'alimentation en eau et/ou en enzymes, avec ou sans modification du débit en substrat lignocellulosique prétraitée , la puissance électrique consommée par le moteur du système d'agitation ramenée à la masse du volume réactionnel restant comprise entre 0,5 et 2kW/tonne,
2) une étape de saccharification du substrat lignocellulosique liquéfié issu de l'étape 1), ladite étape de saccharification étant réalisée en présence d'un microorganisme alcooligène selon un procédé de saccharification et de fermentation simultanées dit procédé SSF.

2. Procédé selon la revendication 1 dans lequel le substrat lignocellulosique prétraitée est mis en contact dans le procédé selon la présente invention à une concentration comprise entre 18 et 40% , avantageusement entre 24 et 40% poids de matière sèche.

3. Procédé selon l'une des revendications 1 ou 2 dans lequel les enzymes sont mises en contact à une concentration comprise entre 10 et 20 mg d'enzymes par grammes de cellulose.

4. Procédé selon l'une des revendications 1 à 3 dans lequel la durée de mise en contact est comprise entre 4 et 8 heures.

5. Procédé selon l'une des revendications 1 à 4 dans lequel l'étape de liquéfaction est mise en œuvre dans un réacteur à alimentation continue au cours de laquelle aucun soutirage du contenu du réacteur n'est effectué.

6. Procédé selon l'une des revendications 1 à 5 dans lequel l'étape de liquéfaction est mise en œuvre dans un réacteur à alimentation continue au cours de laquelle une fraction du volume réactionnel est soutirée de manière à maintenir la masse du volume réactionnel constante.

7. Procédé selon l'une des revendications 1 à 6 dans lequel l'étape de liquéfaction opère à une température comprise entre 40 et 60°C , à un pH compris entre 4 et 6, et à pression atmosphérique.

**Patentansprüche**

1. Verfahren zur Umwandlung eines lignocellulosehaltigen Substrats, wobei es die folgenden Schritte umfasst:

1) einen Schritt des Verflüssigens des Substrats, in welchem 10 bis 40 Gewichts-% an Trockensubstanz des vorbehandelten lignocellulosehaltigen Substrats unter motorgetriebener Rühreinwirkung mit Wasser und mit Enzymen, deren Konzentration im Bereich von 0,1 bis 60 mg an Enzymen pro Gramm an Cellulose liegt, über eine Dauer im Bereich von 1 bis 24 Stunden in Kontakt gebracht werden, wobei im Laufe dieser Zeit zumindest der Wert der elektrischen Leistung gemessen wird, welche von dem Motor des Rührsystems aufgenommen wird, und ein Schritt a) gemäß den folgenden Ausführungen durchgeführt wird, wenn im Laufe der Zeit eine Verringerung dieses Wertes detektiert wird:

a) Erhöhen der Speiseflussrate an vorbehandeltem lignocellulosehaltigem Substrat, mit oder ohne Modifizierung der Flussrate an Enzymen und/oder an Wasser,

und wobei ein Schritt b) gemäß den folgenden Ausführungen durchgeführt wird, wenn im Laufe der Zeit eine Erhöhung dieses Wertes detektiert wird:
b) Erhöhen der Speiseflussrate an Wasser und/oder an Enzymen, mit oder ohne Modifizierung der Flussrate an vorbehandeltem lignocellulosehaltigen Substrat, wobei die elektrische Leistung, welche von dem Motor des Rührsystems aufgenommen wird, im Verhältnis zur Masse des Reaktionsvolumens im Bereich von 0,5 bis 2 kW/Tonne bleibt,
2) einen Schritt des Verzuckerns des verflüssigten lignocellulosehaltigen Substrats, das aus dem Schritt 1) hervorgeht, wobei der Verzuckerungsschritt in Gegenwart eines alkoholerzeugenden Mikroorganismus gemäß einem Verfahren zur gleichzeitigen Verzuckerung und Vergärung durchgeführt wird, welches als SSF-Verfahren bezeichnet wird.

2. Verfahren nach Anspruch 1, wobei das vorbehandelte lignocellulosehaltige Substrat in dem Verfahren gemäß der vorliegenden Erfindung derart in Kontakt gebracht wird, dass seine Konzentration im Bereich von 18 bis 40 %, vorteilhafterweise von 24 bis 40 %, nach Gewicht an Trockensubstanz, liegt.

3. Verfahren nach einem der Ansprüche 1 oder 2, wobei die Enzyme derart in Kontakt gebracht werden, dass ihre Konzentration im Bereich von 10 bis 20 mg an Enzymen pro Gramm an Cellulose liegt.

4. Verfahren nach einem der Ansprüche 1 bis 3, wobei die Dauer des Inkontaktbringens im Bereich von 4 bis 8 Stunden liegt.

5. Verfahren nach einem der Ansprüche 1 bis 4, wobei der Verflüssigungsschritt in einem Reaktor mit kontinuierlicher Speisung durchgeführt wird, wobei im Laufe derselben keinerlei Entnahme des Inhalts aus dem Reaktor erfolgt.

6. Verfahren nach einem der Ansprüche 1 bis 5, wobei der Verflüssigungsschritt in einem Reaktor mit kontinuierlicher Speisung durchgeführt wird, wobei im Laufe derselben eine Teilmenge des Reaktionsvolumens derart entnommen wird, dass die Masse des Reaktionsvolumens konstant bleibt.

7. Verfahren nach einem der Ansprüche 1 bis 6, wobei der Verflüssigungsschritt bei einer Temperatur im Bereich von 40 bis 60°C, bei einem pH-Wert im Bereich von 4 bis 6 sowie bei Atmosphärendruck betrieben wird.

**Claims**

1. Method for converting a lignocellulosic substrate, comprising the following steps:

   1) a step of liquefaction of the substrate in which 10 to 40% by weight of solids of pretreated lignocellulosic substrate are brought into contact, with stirring and by means of a motor, with water and with enzymes at a concentration of between 0.1 and 60 mg of enzymes per gram of cellulose, for a period of between 1 and 24 hours, in which at least the value of the electrical power consumed by the motor of the stirrer system is measured over time, and, if a decrease in said value over time is detected, a following step a) is performed:

   a) the feed flowrate of pretreated lignocellulosic substrate is increased, with or without modification of the flow rate of enzymes and/or of water,

   and if an increase in said value over time is detected, a following step b) is performed:
   b) the feed flowrate of water and/or of enzymes is increased, with or without modification of the flowrate of pretreated lignocellulosic substrate, the electrical power consumed by the motor of the stirring system related back to the mass of the reaction volume remaining between 0.5 and 2 kW/tonne,
   2) a step of saccharification of the lignocellulosic substrate liquefied at the outcome of step 1), said saccharification step being carried out in the presence of an alcohol-producing microorganism according to a process of simultaneous saccharification and fermentation, known as an SSF process.

2. Method according to Claim 1, in which the pretreated lignocellulosic substrate is brought into contact in the method according to the present invention at a concentration between 18 and 40%, advantageously between 24 and 40% by weight of dry matter.

3. Method according to either of Claims 1 and 2, in which the enzymes are brought into contact at a concentration of between 10 and 20 mg of enzymes per gram of cellulose.

4. Method according to one of Claims 1 to 3, in which the contact time is between 4 and 8 hours.

5. Method according to one of Claims 1 to 4, in which the liquefaction step is carried out in a continuously fed reactor during which no drawing off of the content of the reactor is carried out.

6. Method according to one of Claims 1 to 5, in which the liquefaction step is carried out in a continuously fed reactor during which a fraction of the reaction volume is drawn off so as to keep the mass of the reaction volume constant.

7.  Method according to one of Claims 1 to 6, in which the liquefaction step is carried out at a temperature of between 40 and 60°C, at a pH of between 4 and 6, and at atmospheric pressure.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 20100255554 A
- US 2010330638 A
- US 4409329 A

### Littérature non-brevet citée dans la description

- **HODGE et al.** Model-Based Fed-Batch for High-Solids Enzymatic Cellulose Hydrolysis. *Appl.Biochem Biotechnol,* 2009, vol. 152, 88-107
- **ROSGAARD et al.** Effects of Substrate Loading on Enzymatic Hydrolysis and Viscosity of Pretreated Barley Straw. *Appl.Biochem Biotechnol,* 2007, vol. 143, 27-40